# EUROPEAN PATENT APPLICATION

(11) **EP 4 260 795 A1**
(43) Date of publication of application: **18.10.2023**
(21) Application number: 22167639.8
(22) Date of filing: 11.04.2022
(51) Int. Cl.: A61B 3/103, A61B 3/107, A61B 3/028, A61B 3/10, A61B 3/11

(54) **APPARATUS, METHOD AND COMPUTER PROGRAM FOR DETERMINING A REFRACTION OF AN EYE**

(71) Applicant: Ligi Tecnologie Medicali S.r.l., 74100 Taranto (IT)
(72) Inventor: D'IPPOLITO, Giuseppe, 74121 Taranto (IT)
(74) Representative: Bardehle Pagenberg Partnerschaft mbB Patentanwälte Rechtsanwälte

(57) **Abstract**

The present invention relates to an apparatus, a method and a computer program for determining a refraction of an eye. An apparatus for determining an objective refraction of an eye is disclosed wherein the apparatus comprises an optical element configured to compensate an aberration of the eye.

## Description

### 1. Technical field

The present invention relates to an apparatus, a method and a computer program for determining a refraction of an eye.

### 2. Prior Art

Various types of devices and methods are currently known for characterizing or measuring an eye. For example, in the field of optometry or ophthalmology there are several approaches known for determining an objective refraction and/or a subjective refraction of the eye.

For example, determining an objective refraction maybe achieved by guiding light into the eye and measuring the light reflected by the eye. For example, the objective refraction can be determined by means of a retinoscopic analysis, a refractor, an autorefractor and/or an aberrometer, etc. The corresponding objective refraction information may be used as a reference for a further eye examination, for example, the subjective refraction.

Determining subjective refraction may comprise determining parameters of the necessary optical correction means (e.g., optical lenses) for an individual in view of his or her actual visual perception. For example, the subjective refraction can be determined by various lenses functioning as the optical correction means in combination with a reading chart and using feedback by the individual on the visual perception.

However, the known approaches for determining a refraction of an eye may not always lead to optimal results. For example, the known approaches may not consider confounding factors that result from the eye as an optical system itself which could negatively impact the correct determination of the refraction of the eye. Known approaches may thus not always be perfectly suitable for applications that require a reliable information on the refraction of the eye (e.g., for applications such as an eye surgery).

It is thus an object of the present invention to improve determining the refraction of an eye.

### 3. Summary of the invention

The aspects described herein address the above need at least in part.

A first aspect relates to an apparatus for determining an objective refraction of an eye wherein the apparatus comprises an optical element configured to compensate an aberration of the eye.

The aberration to be compensated may comprise (or consist of) a specific (optical) aberration out of a plurality of specific partial (optical) aberrations of the eye which may be present in the optical system of the eye to be examined. For example, the specific (optical) aberration may be associated with a certain component of the eye which is part of the eye's optical system wherein the certain component may cause the specific aberration. For example, the specific aberration may be an aberration that could be difficult to measure and/or that may even falsify the measurement of the total objective refraction of the eye. By implementing the herein described optical element in the apparatus, such aberration may be compensated such that the measurement of the (remaining) aberration is improved. To that regard, the original optical output of the eye's optical system may be modified by the optical element and then measured by the apparatus. The apparatus may thus be configured to measure only a part of the total aberration of the eye (e.g. the total aberration of the eye minus the aberration compensated by the optical element). In some examples, the apparatus may be configured to subsequently determine the objective refraction (e.g. a total aberration) based on the measurement of the modified optical output.

The compensation by the optical element may thus enable selectively suppressing an effect of a specific aberration of the eye. In turn, only the remaining one or more specific aberrations of the eye may be substantially present in the modified or compensated optical output of the eye's optical system during a measurement thereof. The inventor has found out that this can enable an improved determination of the objective refraction of the eye, since a confounding influence of the compensated specific aberration may be substantially suppressed. In turn, this can enable an improved determination of the remaining one or more specific aberrations of the eye. The measurement of the optical output of the eye's optical system for determining the objective refraction can thus be performed more accurately, less noisy and with less (or almost no) crosstalk stemming from the specific aberration that has been compensated.

Surgical procedures that are based on more reliable objective refraction information which are enabled by the herein described invention may lead to improved results for patients since the surgery is based on more accurate information of the eye. For example, a surgical procedure may comprise a corneal ablation to correct a visual ametropia of the eye, wherein a more reliable objective refraction information may lead to an improved surgical parameter and thus an improvement in visual ametropia correction for a patient.

Notably, the specific aberration to be compensated may be predetermined and/or acquired prior to the determination and/or measurement of the objective refraction. In an example, the predetermined specific aberration may be based on a measurement by an ophthalmology and/or optometry apparatus which may not (necessarily) be configured to determine an objective refraction of the eye but, for example, configured to determine the specific aberration based on an according measurement method and/or to determine an aberration from which the specific aberration to be compensated may be readily derived.

In an example, the optical element maybe configured, for a first objective refraction determination, to compensate a first specific aberration, however, for a second objective refraction determination, the optical element may compensate a different specific aberration. For example, the specific aberration to be compensated may be adapted to the individual eye whose objective refraction is to be determined.

It should be noted that the components of the eye's optical system may typically comprise a cornea, a lens, an iris diaphragm (pupil) and a retina of the eye. Further components of the eye's optical system that may also contribute to the one or more aberrations may comprise an anterior chamber, a posterior chamber, an axial length, a vitreous chamber of the eye, as well as any other component of the eye that may influence an optical output of the eye's optical system.

With the eye being an organ, the components of the eye can comprise a biological tissue. However, as it is common to replace and/or adapt certain components of the eye's optical system for medical purposes it should be noted that the invention also covers and refers to implanted and/or adapted components of the eye's optical system that may comprise an artificial (e.g., non-organic) tissue since also the implanted and/or adapted components may cause an aberration of the eye which may in turn influence the determination of the objective refraction, as well. The present invention is thus also suitable to compensate an aberration of the eye that is associated with an implanted and/or adapted component of the eye's optical system.

In an example, the optical element can be configured to compensate a higher order aberration of the eye. The higher order aberration to be compensated may comprise an aberration which is not associated with a lower order aberration of the eye in the field of ophthalmology and/or optometry. The lower order aberration of the eye may comprise the lower order aberrations such as myopia, hyperopia, regular astigmatism and tilting. Typically, these lower order aberrations can be compensated, for example, via a lens with a spherical diopter value, a cylindrical diopter value with a cylinder axis orientation and a prismatic diopter value with a prismatic axis orientation that causes a compensation of the lower order aberration (e.g., this may be a common compensation mechanism present in eyeglasses). However, these lower order compensation mechanisms may not compensate further remaining aberrations of the eye, which may comprise higher order aberrations of the eye. The example considers, however, compensating the remaining (e.g., higher order) aberrations of the eye for an objective refraction determination. The aberration to be compensated may thus comprise a higher order aberration of the eye (or a component thereof, such as the higher order aberration of the cornea) other than second order myopia (e.g., positive defocus), second order hyperopia (e.g., negative defocus), second order astigmatism (e.g., regular astigmatism) and first order of tilting of the eye (or the cornea). In some examples, the aberration to be compensated may only comprise a higher order aberration of the eye (or a component thereof, such as the higher order aberration of the cornea) other than second order myopia (e.g., positive defocus), second order hyperopia (e.g., negative defocus), second order astigmatism (e.g., regular astigmatism) and first order of tilting of the eye (or the cornea). In some examples, the aberration to be compensated may comprise the entire higher order aberration of the eye and/or of the cornea other than second order myopia (e.g., positive defocus), second order hyperopia (e.g., negative defocus), second order astigmatism (e.g., regular astigmatism) and first order of tilting of the eye and/or of the cornea.

In some examples, the apparatus and/or the optical element can be configured to compensate a third order and/or an even higher order aberration of the eye, without (necessarily) compensating a spherical diopter value and/or a cylindrical diopter value with a cylinder axis orientation and/or a prismatic diopter value with prismatic axis orientation.

For example, the order of aberration of the eye may constitute the order of aberration according to the Zernike polynomials which are well known in the field. To that regard, a Zernike polynomial may be described as Zₙ wherein the subscript n specifies the order of aberration. In this example, the third order aberration to be compensated constitutes an aberration which corresponds to a Zernike polynomial according to Zₙ with n = 3. The (even) higher order aberration may comprise an aberration of at least the fourth order according to the Zernike polynomials (e.g., with n ≥ 4 in a Zernike polynomial according to Zₙ). However, the higher order aberration to be compensated is not limited to a definition according to the Zernike polynomials. As the skilled person is aware, there may be a wide range of other ways to describe aberrations (e.g., such as higher order aberrations). The inventive concept thus also considers compensating various other types of a higher order aberration (e.g., usually termed HOA). In an example, the higher order aberration may be caused by biological damage of the eye's optical system (e.g., scar tissue, injury, surgical complication and/or an eye disease) and/or any other biological irregularity of the eye's optical system (e.g., a corneal irregularity, an eye lens irregularity, etc.).

In an example, the optical element can be configured to compensate a first order aberration and/or a second order aberration of the eye. For example, the second order aberration may comprise myopia, hyperopia and regular astigmatism. The compensation of the second order aberration may thus comprise compensation by the optical element based on a spherical diopter value and/or a cylindrical diopter value with a cylinder axis orientation. The first order aberration may comprise a tilt of the eye's optical system which may be compensated via a prismatic diopter value with a prismatic axis orientation. The tilt may be compensated via the optical element by adapting the optical output of the eye via a horizontal and/or vertical compensating tilt, for example. Also, the herein mentioned first order aberration and/or second order aberration may constitute the order of aberration according to the Zernike polynomials which are well known in the field. In this example, the aberration to be compensated may for example comprise an aberration which corresponds to a Zernike polynomial according to Zₙ with n = 1 and/or n = 2. For example, the first order to be compensated by the optical element may comprise a tilt as defined by a Zernike polynomial of the first order. In another example, the second order aberration to be compensated by the optical element may comprise an astigmatism (e.g., an oblique and/or a vertical astigmatism) and/or a defocus, as defined by a Zernike polynomial of the second order.

In a further example, the optical element can be configured to compensate a lower order aberration of the eye (e.g., as defined in the field of ophthalmology and/or optometry). To that regard, the lower order aberration may comprise a first order and/or second order aberration of the eye according to the Zernike polynomials as described herein but no higher order aberrations. In an example, the lower order aberration to be compensated may, however, also be defined as an aberration of the eye which comprises the second order myopia (e.g., positive defocus), second order hyperopia (e.g., negative defocus) and/or second order astigmatism (e.g., regular astigmatism) of the eye but not the first order aberration, for example.

In an example, the optical element can be configured to compensate an aberration of a cornea of the eye. For example, the aberration of the cornea may comprise a higher order aberration of the eye as described herein. The (specific) aberration to be compensated by the optical element may thus for example comprise a higher order aberration of the cornea. The higher order aberration of the cornea may in particular comprise a third order and/or one or more (e.g. all) even higher order aberrations as described herein which is induced by the cornea of the eye (whereas other aberrations of the eye may not be compensated).

In some examples, the optical element and/or the apparatus may be configured to compensate third and higher order aberrations of the cornea, but spherical and cylindrical aberration, as well as any aberrations caused by other elements of the eye may not be compensated. Since the cornea typically provides the strongest contribution to higher order aberrations caused by irregular corneal shapes, compensating specifically this effect may enable a particularly strong reduction of measurement artefacts, such that the remaining aberration of the eye may be measured more reliably.

The aberration of the cornea to be compensated may in general be derived from one or more parameters of the cornea and/or a combination of parameters of the cornea. For example, a parameter of the cornea that may invoke a respective aberration of the cornea may comprise a corneal surface irregularity of an anterior and/or posterior corneal surface shape, an anterior and/or posterior corneal surface topology, a corneal thickness (variation and/or distribution), a corneal refractive index (variation and/or distribution), the cornea's geometry, etc.

In an example, the optical element can be configured to compensate the aberration of the cornea based at least in part on a topographic and/or a tomographic information of the cornea. The topographic and/or tomographic information may comprise a dataset of the cornea (e.g., a map of the cornea) that indicates for various positions of the cornea a respective corneal information (e.g., a height, a corneal radius, a curvature steepness, an axial/sagittal curvature, an elevation, a corneal thickness, a refractive power, an axial power, a corneal pachymetry information etc.), as is well known in the field. The topographic and/or tomographic information may also comprise an anterior and/or posterior corneal surface shape, as well as an anterior and/or posterior corneal surface topology. The respective corneal information may be indicated, for example, for positions at the anterior and/or posterior surface of the cornea. The compensation of the corneal aberration by the optical element can be further based on determining the aberration of the cornea based on the topographic and/or tomographic information of the cornea. As is known in the field, the topography/tomography of the cornea may have an irregular shape causing light, for example, to be refracted in an irregular way such that a specific aberration, which is associated with the corneal topography/tomography, is induced for the eye's optical system. The inventor, however, considered that such an induced specific aberration stemming from the irregularity of the tomography/topography of the cornea may negatively impact the determining of the remaining objective refraction of the eye (e.g., the determining of the remaining aberrations of the eye). Therefore, in an example of the invention, such an irregularity of the tomography/topography of the cornea may be considered based on the topographic/tomographic information of the cornea and its induced aberration effect may be suppressed. To that regard, this induced aberration by the cornea's shape may be compensated by the optical element such that it is corrected for the eye's optical system. The apparatus may thus measure the remaining objective refraction in a highly accurate manner. By adding the measured remaining objective refraction and the compensated aberration (e.g. by the apparatus), the total objective refraction can be obtained (e.g. by the apparatus).

For example, the higher order aberration of the cornea may be derived (e.g. by the apparatus) based at least in part on a topographic and/or a tomographic information of the cornea, and the optical component may be configured (e.g. by the apparatus) to compensate the higher aberration of the cornea derived in that manner.

In an example, the apparatus further comprises means for receiving the topographic and/or the tomographic information. The apparatus may further comprise means for determining (e.g., calculating) the (higher order) aberration of the cornea based on the received topographic and/or tomographic information. In another example, the apparatus comprises means for receiving the (higher order) aberration of the cornea which is based on the topographic and/or tomographic information thereof.

In an example, the optical element can comprise an adaptive optics and/or an adaptive mirror. The adaptive optics and/or mirror may comprise a deformable optics and/or deformable mirror whose surface/s can be deformed to adapt the properties of light which is refracted and/or reflected by the deformable optics and/or the deformable mirror. The adaptive optics and/or adaptive mirror may be used to compensate the aberration of the eye by the optical element, as outlined herein. To illustrate an example, the deformable optics and/or the deformable mirror may compensate the aberration of the cornea based at least in part on a corneal topography and/or tomography map.

In an example, the optical element can comprise a first optical component configured to compensate the first order aberration, a second optical component configured to compensate the second order aberration and/or a third optical component configured to compensate a higher order aberration of the eye. The optical element may thus comprise various optical components which are constructed to compensate a specific order of the aberration of the eye's optical system. This may reduce the system complexity of the apparatus since each optical component may be optimized for a specific aberration which in turn may lead to an overall easier compensation mechanics, since not every optical component may be advantageous or suitable for compensating a plurality of specific aberrations.

In an example, the apparatus may further comprise a detector for detecting an electromagnetic wave reflected by the eye and/or a divider element positioned between the detector and the eye, wherein the divider element is configured such that the electromagnetic wave is divided into a plurality of wave components. The detector may thus enable the apparatus to measure the electromagnetic wave which was shaped by the eye's optical system, as well as the optical element. To that regard, the reflection of the electromagnetic wave may have taken place at any point and/or plane of the eye's optical system such that an aberration of the eye (e.g., a specific aberration of a component of the eye's optical system) may have been invoked onto the reflected electromagnetic wave. For example, the electromagnetic wave may have been (substantially) reflected at the retina. The reflected electromagnetic wave may constitute the optical output of the eye's optical system as stated herein. Notably, the electromagnetic wave may have been actively guided into the eye's optical system for a controlled reflection thereof by the eye to determine the objective refraction of the eye. The apparatus may thus also comprise an emitter to emit the electromagnetic wave and means for guiding the electromagnetic wave onto a desired reflection point or plane of the eye. The electromagnetic wave may comprise an electromagnetic wave of various wave lengths. To that regard, the wavelength of the electromagnetic wave may comprise a wavelength usually associated with light (e.g., a wavelength that constitutes UV-light, visible light and/or infrared light, or a combination thereof).

The (at least one) divider element may be configured such that a single beam or a bundle of beams of the reflected electromagnetic wave impinging onto the divider element is/are separated into a plurality of wave components that may comprise separately defined ray bundles that may subsequently impinge onto the detector in a spatially separated manner. The apparatus may, in particular, be configured to implement a wavefront sensor via the divider element and detector assembly. For example, the divider element may comprise a lens array and/or a lenslet array wherein the detector may comprise a sensor array (e.g., to implement a Shack-Hartmann wavefront sensor). The divider element may also comprise a pyramidal prism (e.g., to implement a pyramid wavefront sensor). In another example, the apparatus may be configured to implement various other wavefront sensors (e.g., a wavefront curvature sensor, an interferometer-based wavefront sensor, etc.). The apparatus may be further configured to determine the objective refraction of the eye based on the detected wavefront of the reflected electromagnetic wave according to known principles in the field. However, the invention enables a correction of the reflected electromagnetic wave before it impinges on the detector (or divider element) since the reflected wave may be further shaped by the optical element that can compensate a (specific) aberration of the eye that may negatively interfere with the measurement of the reflected electromagnetic wave. By reducing the effect of the (specific) aberration onto the measurement the determining of the objective refraction maybe significantly improved.

In an example, the divider element may be configured such that at least one of the wave components (or each wave component) comprises a portion of the electromagnetic wave originating from a corresponding region on a surface of a cornea of the eye. The optical element may be configured to compensate the aberration such as to reduce an amount of the electromagnetic wave in the at least one of the wave components (or in each wave component) that originates from a different region of the cornea; and/or such as to increase the portion of the electromagnetic wave originating from the corresponding region of the cornea comprised by the at least one of the wave components. For example, for determining the correct objective refraction by the apparatus it is assumed that the divider element is positioned such that a wave component W1 detected at the detector comprises substantially the portion of the electromagnetic wave originating from a particular (corresponding) region R1 of the cornea. In this example, the wave component W1 should thus map to region R1. However, due to a (specific) aberration of the eye (e.g., a (higher order) corneal aberration), an amount of the electromagnetic wave originating from a different region R2 maybe significantly deflected such that it impinges onto the divider element such that its wave component W2 may crosstalk into the wave component W1 that is initially assumed to comprise substantially the portion of the electromagnetic wave originating from region R1. Hence, with no compensation of the aberration present, the wave component W1 may comprise the portion of the electromagnetic wave originating from region R1 (as intended), however, also of region R2 (as not intended) such that a measurement error is introduced which may lead to a less accurate determination of the objective refraction. The optical element, according to the invention, may thus enable a correction mechanism by shaping the electromagnetic wave by compensation of the aberration of the eye such that the herein described crosstalk of the parasitic electromagnetic wave portion originating from a different (non-corresponding) region (in this example R2) is reduced. Reduction of crosstalk may similarly be achieved by increasing the amount of the electromagnetic wave originating from (the corresponding) region R1 via the compensation of the optical element, e.g. by compensating the aberration such as to reduce the portion of the electromagnetic wave originating from (the corresponding) region R1 that is deflected away and thus detected as part of a wave component that corresponds to a different region (e.g. R2).

To illustrate another example, it may also be conceivable that for determining an objective refraction it has to be assumed that a specific part of the divider element (e.g., a specific lenslet of a lenslet array) maps with a corresponding region R1 of the cornea wherein it may be further assumed that the detected wave component W1 originating from the specific part of the divider element only comprises the portion of the electromagnetic wave originating from the corresponding region R1. However, without the inventive compensation, the herein described crosstalk may, also in this example, cause a portion of an electromagnetic wave originating from a different (non-corresponding) region R2 to be (e.g., partly) deflected onto the specific part of the divider element (e.g., the specific lenslet of the lenslet array) such that the detected wavefront W1 comprises portions of the electromagnetic waves originating from region R1 and R2. Also in this example, the optical element, according to the invention, may enable a correction mechanism by shaping the electromagnetic wave (e.g., prior to impinging onto the divider element) by compensating the aberration of the eye such that the herein described crosstalk of the parasitic electromagnetic wave portion originating from a different (non-corresponding) region is reduced.

In an example, the optical element maybe configured to compensate the aberration such that the at least one of the wave components (or each of the wave components) and/or the part of the divider element from which it emerges has a one-to-one correspondence to the corresponding region of the cornea. For example, the one-to-one correspondence may comprise that the crosstalk of the parasitic electromagnetic wave portion (as described herein) is completely reduced. Hence, the at least one of the wave components may comprise substantially the complete information of the portion of the electromagnetic wave when originating from its corresponding region in the cornea without any interfering noise. The complete information may constitute that the characteristics of the portion of the electromagnetic wave, when originating from the cornea, can be deducted based on the at least one of the wave components. Notably, this may enable to determine the part of the wavefront of the electromagnetic wave in the location of the corresponding region with a one-to-one correspondence.

As stated herein, the apparatus may be configured to comprise the detector and the divider functioning as a wavefront sensor, wherein the determination of the objective refraction may be based on the wavefront of the electromagnetic wave when originating from the cornea.

In a further example, the aberration is compensated by the optical element such that substantially all detected wave components have a one-to-one correspondence to their corresponding regions of the cornea. This may enable to suppress the effect of the aberration substantially to a maximum degree to enable a measurement that is substantially free of the (specific) aberration noise which in turn may improve determining the objective refraction.

In a further example, the optical element may be configured to compensate the aberration such that a portion of the electromagnetic wave that originates from a specific region of the cornea when impinging on the divider element has a one-to-one correspondence to the portion of the electromagnetic wave originating from the specific region.

In an example, the apparatus can further comprise an optical path for determining a subjective refraction associated with the eye, wherein the optical element is arranged in the optical path. The apparatus may thus also be configured to function in addition as an apparatus to perform a subjective refraction determination wherein the compensation capabilities of the optical element (as described herein) may be used analogously for performing the subjective refraction examination. The optical path of the subjective refraction may, for example, be partly shared with an optical path used for determining the objective refraction of the eye (as described herein). The optical element may thus also be further configured to compensate a specific aberration in the field of vision of an examined individual (i.e., into the optical path for determining a subjective refraction).

In particular, the optical element may be configured to compensate a lower and/or higher order aberration as described herein for the objective refraction into the optical path for determining the subjective refraction. The optical element may thus not be exclusively configured to shape an optical output (e.g., to compensate the specific aberration) of the eye's optical system for determining an objective refraction but also to shape an optical input to the eye's optical system (e.g., compensating the specific aberration). To that extent, the optical path for determining a subjective refraction may comprise a screen (e.g., in form of an electronic display, or a physical poster/board) to display a visual chart in the optical path for an individual to perform a subjective refraction. With the shaping of the optical input to the individual's eye by the optical element, higher order aberrations as well as lower order aberrations may thus be compensated, e.g. with the herein described capabilities of the optical element. This example maybe beneficial as the objective and subjective refraction examination is made available on a single apparatus. Moreover, the system complexity maybe reduced as the need for two separate apparatuses with separate optical components for optical beam shaping may be abstained from.

A second aspect relates to an apparatus for determining a subjective refraction of an eye, comprising: an optical element configured to compensate an aberration of the eye, wherein the aberration comprises a higher order aberration of the eye.

The apparatus of the second aspect may comprise one or more parts of the apparatus of the first aspect, however, it may not necessarily be configured to determine an objective refraction of the eye. As the apparatus of the second aspect may compensate a higher order aberration of the eye, it may also be viewed as a "higher order phoropter". As is common in the field, usually the subjective refraction is determined based on compensating the lower order aberrations of the eye (e.g., the second order aberrations, such as myopia, hyperopia, regular astigmatism). However, the apparatus of the second aspect, may enable analyzing the effects or correction of higher order aberrations for an individual's subjective refraction (e.g., for a third order and/or even higher order aberration as described herein). The complexity of the visual perception of an individual may thus be considered to a fuller extent. For example, the results of the subjective refraction via the apparatus according to the second aspect (i.e., the "higher order phoropter") may thus improve the assessment of a surgical procedure for the eye.

The elements described for the second aspect may also apply for the subjective refraction determination described as an option for the apparatus of the first aspect.

In an example, the apparatus according to the first and/or second aspect may further comprise means for controlling a diameter of an iris diaphragm of the eye by illuminating the iris of the eye. For example, the means for controlling maybe configured to set a predetermined diameter of the iris diaphragm for a subsequent examination of the eye. In particular, the diameter may be set for determining the objective and/or subjective refraction, respectively. The level of illumination by the means for controlling may cause the iris diaphragm of the eye to adapt to a certain diameter that can be thereby set or controlled. The level of illumination may for example comprise a level of an intensity, a power and/or a brightness of the illumination. The illumination itself may comprise an electromagnetic wave with a wavelength that corresponds to light in the visible range such that the eye may be triggered to accordingly adapt the iris diaphragm.

In a further example, the means for controlling may be further configured to measure the diameter of the iris diaphragm. This may enable to check which iris diaphragm diameter was set via the illumination of the eye. Moreover, this may enable to implement a control loop where the illumination is controlled in response to the measured diameter of the iris diaphragm to implement the setting of a desired iris diaphragm diameter. The measurement of the diameter may be based on a second illumination of the eye which may comprise an electromagnetic wave with a wavelength that corresponds to light in the invisible range (e.g., the infrared light range, e.g., light with a wavelength above 700 nm).

In a further example, the means for controlling may be configured such that determining the (objective and/or subjective) refraction by the apparatus according to the first and/or second aspect may be performed at the predetermined diameter of the iris diaphragm. This may ensure that each measurement for the (objective and/or subjective) refraction according to the invention may be based on a defined iris diaphragm. Hence, a more reliable determination of the refraction of the eye may be performed as the factor of a varying (or unknown) iris diaphragm is reduced. Moreover, the effect of the iris diaphragm diameter on the eye's optical system may thus be tuned to a specific value such that various measurements (e.g., of the subjective/objective refraction) may be more comparable. Further, the refraction of the eye can be performed for a particular set of iris diaphragm diameters to assess the characteristics of the (objective and/or subjective) refraction to a fuller extend. For example, the (objective and/or subjective) refraction maybe analyzed for a darker surrounding (e.g., by a low-level illumination that may lead to a relatively increased iris diaphragm) and for a brighter surrounding (e.g., by a high-level illumination that may lead to a relatively reduced iris diaphragm).

A third aspect relates to a method for determining an objective refraction of an eye, comprising: compensating an aberration of the eye by an optical element. By compensating a (specific and/or partial) aberration of the eye, the objective refraction may be determined more accurately, as outlined herein.

In an example, the method may further comprise obtaining and/or measuring topographic and/or a tomographic information of a cornea of the eye, wherein the compensating comprises compensating an aberration of the cornea based on the topographic and/or a tomographic information.

In an example, the compensating may further comprise compensating a first order, a second order, a third order and/or even a higher order aberration of the eye. In some examples, the compensating may comprise compensating third and/or higher order aberrations of the eye, e.g. third and/or higher order aberrations of the cornea, whereas spherical and regular cylindrical aberrations as well as aberrations caused by other elements of the eye are not compensated.

In an example, the step of compensating may comprise compensating a corneal refractive aberration of the eye.

In an example, the method may further comprise determining an aberration of the eye based on a measurement with the compensated aberration (of the optical element, as described herein). In an example, the method may then further comprise compensating also the determined aberration by means of the optical element. The measurement may then be repeated such that the still remaining aberration (if any) can be measured, and so forth. Hence, an iterative approach can be used, in which the total aberration of the eye is compensated step-by-step by means of the optical element until the output of the measurement device does not indicate any remaining aberration anymore.

A fourth aspect relates to a method for determining a subjective refraction of an eye comprising compensating a higher order aberration of the eye by an optical element.

A fifth aspect relates to a computer program comprising instructions that when executed by a computer or an apparatus according to one of the herein described aspects causes the computer or the apparatus to perform a method according to one of the herein described aspects. For example, the apparatus according to the first aspect (or second aspect) as described herein may comprise computing means (e.g., a computer, a processor, etc.) wherein the computer program may cause the apparatus of the first aspect (or the second aspect) to perform one of the according methods described herein.

It is noted that the method steps as described herein may include all aspects described herein, even if not expressly described as method steps but rather with reference to an apparatus (or device or system or computer). Moreover, the devices and computer programs as outlined herein may include means for implementing all aspects as outlined herein, even if these may rather be described in the context of method steps.

Whether described as method steps, computer program and/or means, the functions described herein may be implemented in hardware, software, firmware, and/or combinations thereof. If implemented in software/firmware, the functions may be stored on or transmitted as one or more instructions or code on a computer-readable medium (e.g., on the apparatus according to one of the herein described aspects). Computer-readable media include both computer storage media and communication media including any medium that facilitates transfer of a computer program from one place to another. A storage medium may be any available media that can be accessed by a general purpose or special purpose computer. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor.

### 4. Brief description of the drawings (Stop)

- Fig. 1: shows a block diagram of an exemplary apparatus according to the invention.
- Fig. 2: is a schematic representation of an Objective Refraction Channel (ORC) of an exemplary apparatus according to the invention, to determine an objective refraction of a patient's eye.
- Fig. 3: shows a schematic representation of an electromagnetic wave reflected at a retina of an eye and exiting a cornea of the eye.
- Fig. 4: shows a magnified view of a portion of Fig. 3.
- Fig. 5: is a schematic representation of a Dynamic Pupillometric Channel (DPC) of an exemplary apparatus, to analyze and control the iris diaphragm diameter (20).
- Fig. 6: is a schematic representation of a Subjective Refraction Channel (SRC) of an exemplary apparatus, to analyze the patient's subjective refraction.
- Fig. 7: is a schematic representation of an exemplary apparatus comprising ORC, SRC, and DPC channels.
- Fig. 8: is a schematic representation of the iris diaphragm diameter of the eye (E).

### 5. Detailed description of the drawings

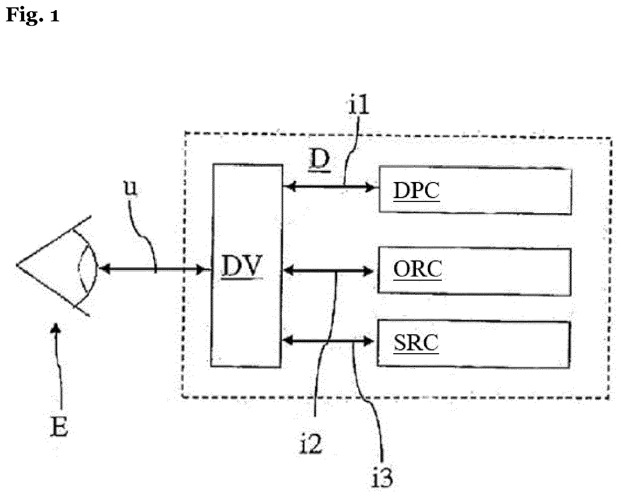

In Figure 1, a schematic representation of an eye E is depicted with respect to a block diagram of an exemplary apparatus D according to the invention. The exemplary apparatus D may enable to determine a refraction of an eye of an individual (e.g., a patient) and may be configured to implement various types of measurements. The exemplary apparatus may comprise an optical divider DV that may serve to connect (or switch) various optical paths of the exemplary apparatus with each other (or onto each). The optical divider may comprise one or more partially reflecting mirrors and/or a lozenge optical divider. For example, there may be a main optical path between the optical divider DV and the eye. The optical divider may be configured such that the optical divider DV connects a first optical path ii, a second optical path i2 and/or a third optical path i3 with the main optical path u. The optical divider may thus divide the main optical path u into separate optical paths (e.g., the first optical path ii, the second optical path i2, the third optical path i3, etc.). The herein described optical paths may be bidirectional such that an electromagnetic wave (e.g., light) may travel in both directions of the optical paths. For example, light may be guided onto the eye via one or more optical paths and light may also be guided from the eye onto the divider element to the various other optical paths (e.g., ii, i2 and/or i3) of the exemplary apparatus D.

In some examples, optical divider DV may be omitted and only path i2 or only path i3 may be present.

The optional first optical path ii may be connected to an optional apparatus unit that is configured to control the diameter of the iris diaphragm wherein said apparatus unit (as well as the optical path of said apparatus unit) may be referred to as the dynamic pupillometric channel DPC (or DPC unit). The second optical path i2 may be connected to an apparatus unit that is configured to determine the objective refraction of the eye wherein said apparatus unit (as well as the optical path of said apparatus unit) may be referred to as the objective refraction channel ORC (or ORC unit). The third optical path i3 may be connected to an apparatus unit that is configured to determine the subjective refraction of the eye wherein said apparatus unit (as well as the optical path of said apparatus unit) may be referred to as the subjective refraction channel SRC (or SRC unit).

Notably, in Fig.i, the objective refraction channel ORC and the subjective refraction channel SRC are arranged on different and separate optical paths only for an easier comprehension of the invention. However, elements of the optometric measurement channels SRC and/or ORC can also rest on the dynamic pupillometric channel DPC (or DPC unit) and vice-versa. The herein described different measurement channels (or apparatus units) may also share parts of a same optical path.

In an example, the exemplary apparatus comprises all three of the herein described units, namely the DPC unit, the ORC unit and the SRC unit. However, as stated herein, the apparatus according to the invention may comprise any combination of the three units and may not be limited to a certain number of units. For example, the apparatus D may solely comprise the ORC unit (and may thus solely be used to determine an objective refraction). In another example, the apparatus D may solely comprise the SRC unit (and may thus solely be used to determine a subjective refraction). In a further example, the apparatus may comprise the ORC unit and the SRC unit such that an objective, as well as a subjective refraction can be determined via the apparatus D. In addition, the apparatus may also comprise the DPC unit in combination with the ORC unit and/or the SRC unit such that the determination of the objective and/or subjective refraction may be accompanied by the functionality of the DPC unit. To that regard, the DPC unit may enable to measure and control the diameter of the iris diaphragm while an optometry measurement is executed via the ORC unit and/or SRC unit.

Notably, the DPC unit, the ORC unit and/or the SRC unit may be separate apparatuses and may thus be coupled by the optical divider DV to form a system that functions as if it were a single apparatus according to the invention. Alternatively, they may form an integrated system.

In the following, the units of the apparatus and their possible interactions and combination are explained.

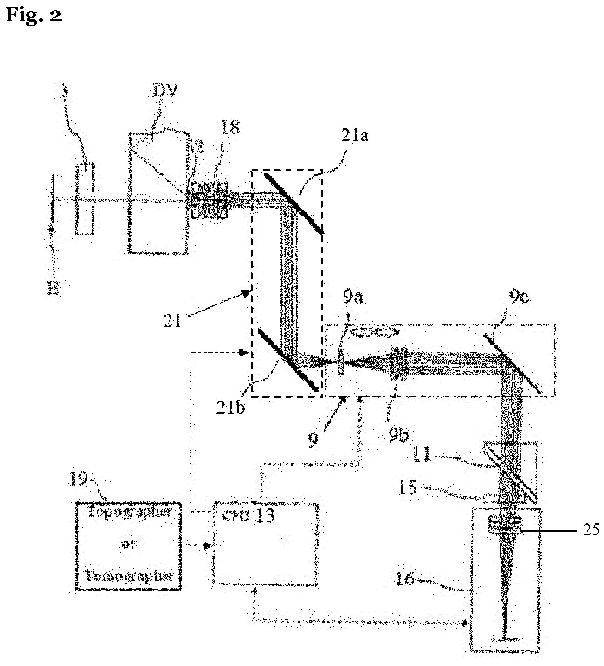

Fig. 2 shows a schematic representation of an exemplary Objective Refraction Channel ORC (or an ORC unit) of an exemplary apparatus according to the invention, to determine an objective refraction of an individual's eye. The objective refractometric measurement of the eye E may be executed in far distance vision condition and/or in accommodated near distance vision condition which may be achieved by means of the apparatus described further herein. To determine an objective refraction, initially a light beam may be guided into the eye E such that it is reflected by the retina of the eye and may subsequently pass through the eye's lens and the cornea of the eye. The part of the light beam that is guided into the eye may be referred as the optical input to the eye's optical system wherein the part of the light beam that is reflected at the retina may be referred to as the optical output of the eye's optical system. The reflected light beam (i.e., the optical output) exiting the cornea may be subsequently measured to determine the eye's objective refraction. Fig. 2 shows to that regard schematically the eye E and the objective refraction path i2 that the light beam (also referred to herein as the objective refraction OR light beam) reflected at the retina is guided onto.

As the exemplary apparatus may comprise various other optical components for analyzing the eye, the OR light beam may initially pass various optical components (such as an LED assembly 3 and/or an optical divider DV) that may not significantly interfere with the OR light beam. Subsequently the OR light beam may pass a focusing lens 18 which may comprise various lenses to focus and/or collimate the OR light beam. Subsequently, the OR light beam may be shaped by an optical element as described herein to compensate a (specific) aberration of the eye. The optical element may comprise various components to shape the OR light beam (however, the herein described optical element may also be comprised of a single optical component). In the example of Fig. 2, the optical element may comprise a group of tilting mirrors 21 that may comprise a first tilting mirror 21a and a second tilting mirror 21b. The optical element may further comprise an optical correction group 9. The optical correction group 9 may comprise a first correction unit 9a, a second correction unit 9b and a third correction unit 9c. The components of the optical element may according to the invention correct the light of the OR light beam such that the (specific) aberration of the eye is not substantially present anymore and thus compensated. The OR light beam exiting the optical element may thus be a corrected OR light beam (having the (specific) aberration suppressed). The corrected OR light beam may be subsequently guided through various other optical components (e.g., an optical divider 11, a bandpass filter 15) and eventually may enter a wavefront analyzer 16. The wavefront analyzer 16 may comprise a divider element, e.g. a multi-lens optics, e.g., in the form of a lenslet array 25, and the herein described detector for detecting the (corrected) OR light beam. The wavefront analyzer may also comprise a pyramidal optic (as the divider element) and the herein described detector to implement a pyramidal wavefront sensor, for example. The compensation by the optical element may ensure that the OR light beam impinging onto the wavefront analyzer 16 (e.g., onto its multi-lens optics, its lenslet array, its pyramidal optic and/or its detector) is a corrected OR light beam that does not comprise a (specific) aberration of the eye. For example, this may ensure that a noise of the (specific) aberration that may alter the measurement is reduced. In another example, the compensation by the optical element may also enable determining a (specific) aberration of the eye.

In the following, aspects of the exemplary optical element and its components are described.

The tilting mirrors 21a and 21b of the tilting mirrors group 21 may be used to correct components Kx and Ky of an angle K of the OR light beam exiting the eye E. The mirrors 21a and 21b may be preferably motorized and controlled by a control unit 13 of the apparatus which may enable a dynamical compensation of the components Kx and Ky of the angle K. For example, the tilting mirrors 21a and 21b may compensate the first order aberration of the eye which may be a vertical tilt and/or a horizontal tilt (e.g., according to the first order Zernike polynomials).

The optical correction group 9 may be motorized and comprise a series of lenses and/or mirrors to compensate a second order aberration (e.g., a second order spherical and/or cylindrical refractive aberration), as well as a high order aberration of the eye for the OR light beam. The optical correction group 9 may be controlled by the control unit 13 to dynamically compensate the objective refraction of the eye E measured by the wavefront analyzer 16.

The optical unit 9a may enable to compensate or correct a second order spherical aberration (e.g., a spherical refractive error, e.g., a defocus). This may be achieved with a motorized spherical optic controlled by the control unit 13. For example, the optical unit 9a may comprise a lens that can be shifted along the optical path of the OR light beam to correct a second order spherical aberration of the eye E that was imparted onto the OR light beam. In a further example, the compensation by the optical unit 9a may be achieved by a spherical optic with an adaptive profile controlled by the control unit 13.

The optical unit 9b may enable to compensate or correct a second order cylindrical aberration of the eye E that was imparted onto the OR light beam (e.g., a cylindrical refractive error, e.g., regular astigmatism). This may be achieved with a motorized cylindrical optic that compensates optical power and angle stemming from the second order cylindrical aberration by means of the control unit 13 (the optical unit may thus compensate a cylindrical diopter value with a certain cylinder axis value). In a further example, this may be achieved by a cylindrical optic having an adaptive profile that compensates optical power and angle stemming from the second order cylindrical aberration by means of the control unit 13. Notably, the optical unit 9b may comprise any correction mechanism known in the field for correcting a second order cylindrical aberration of the eye.

The optical unit 9c may enable to compensate or correct higher order aberration of the eye. The higher order aberration may comprise a third order or even higher order aberration of the eye (or the cornea) as described herein. The optical unit 9c may comprise an adaptive optic/mirror 9c which can be used to compensate one or more higher order aberrations of the eye E. This may be achieved by the adaptive optic/mirror 9c which may be shaped, by means of a set of piezoelectric actuators that may be controlled by control unit 13 such that one or more higher order aberrations of the eye E that were imparted onto the OR light beam are compensated. The adaptive optic/mirror 9c may comprise a deformable mirror that can be adjusted via the set of piezoelectric actuators.

In an example, the adaptive optic/mirror 9c may be shaped to compensate the higher order corneal refractive aberrations of the cornea E which were detected by means of a corneal topographer and/or tomographer 19. The corneal refractive aberrations usually represent the highest percentage of the total higher order aberrations of the eye and may thus impart the most effect onto the OR light beam.

In another example, the control unit 13 may control the shape of the adaptive optic/mirror 9c to also compensate the first order aberration of the eye E and/or the spherical second order aberration of the eye E and/or the cylindrical second order aberration of the eye E. The adaptive/mirror optic 9c may thus partially or completely replace the respective functionality of the tilting mirrors 21a and 21b and/or the optical unit 9a and/or the optical unit 9b.

In an example of the present invention, the control unit 13 may receive the (higher order) corneal refractive aberration of the eye E measured by means of a corneal topographer or tomographer 19 and may preventively control the shape of the adaptive optic/mirror gc to compensate the (higher order) corneal refractive aberration of the eye E. The measured wavefront data may thus be corrected from the effect of the (higher order) corneal refractive aberrations of the eye. Subsequently, the control unit 13 may detect the compensated wavefront data measured by the wavefront analyzer 16. Based on the measured wavefront data, the aberrations of the eye without the effect of the corneal refractive aberration may be determined. However, based on the measured wavefront data, the control unit 13 may further iteratively control the other beam shaping optics in the apparatus to compensate the remaining aberrations of the eye present in the OR light beam (e.g. via the optical element and its components). For example, the control unit 13 may further control the mirrors 21a and 21b to compensate the first order aberration of the eye E and/or the optical unit 9a to compensate the second order spherical aberration of the eye E and/or the optical unit 9b to compensate the cylindrical second order aberration of the eye E and/or the adaptive optic/mirror 9c to compensate the (remaining) higher order aberrations of the eye E. The remaining wavefront may thus be substantially free of the eye's aberrations. Based on the further compensation the objective first, second and (remaining) higher order aberrations of the eye E may be determined with a high level of accuracy (e.g., in the specific conditions of far and/or near vision distance, e.g., with a preselected diameter of an iris diaphragm 20 which was set by the DPC unit).

In an example, the control unit 13 may control the shape of the adaptive optic/mirror 9c to compensate not only the (remaining) higher order aberrations but also the first order aberration of the eye, the spherical second order aberration of the eye and/or the cylindrical second order aberration of the eye.

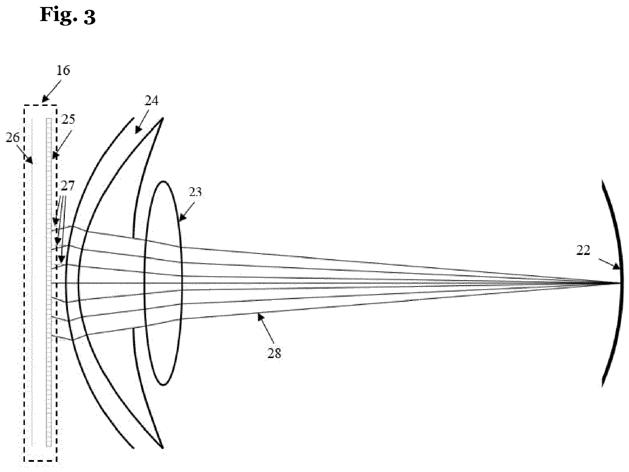

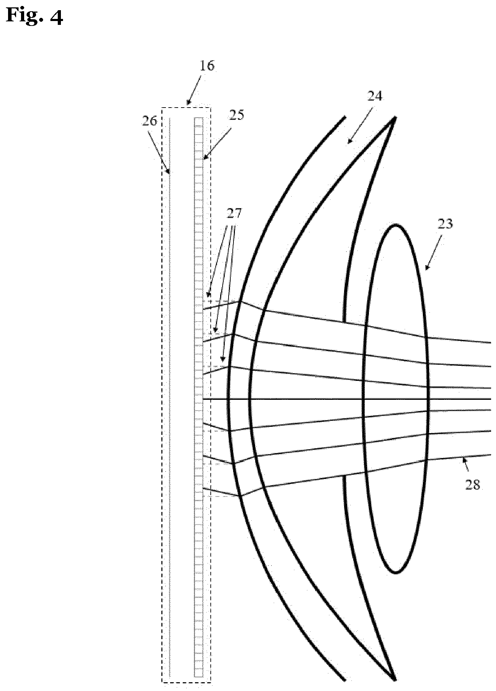

Moreover, the herein described correction of the aberration (e.g., in particular correcting the corneal refractive aberrations) may ensure a one-to-one correspondence between the wavefront when exiting the cornea of the eye and when impinging on the divider element. This is for example, schematically represented in Fig. 3 that depicts an electromagnetic wave 28 being reflected at the retina 22 of the eye and subsequently passing the eye's lens 23 and the cornea 24 and impinging on the divider element and ultimately a detector 26 of wavefront analyzer 16. In this example, the divider element is a lenslet array 25. When exiting the cornea, the reflected electromagnetic wave may be modeled via various light beams 27 that extend from the cornea to the lenslet array 25. A light beam 27 impinging on a lenslet of the lenslet array 25 may thus originate from a corresponding region of the cornea. To that regard, Fig. 4 shows an enlarged view of the light beams 27 impinging on the lenslets of the lenslet array. However, due to corneal refractive aberrations (or any other higher order aberration) the light beam 27 may be highly deflected such that it does not impinge on its corresponding lenslet of the lenslet array. In another example, the light beams 27 may be deflected by the corneal refractive aberrations in an irregular way such that the electromagnetic wave impinging on the lenslet array 25 does not correspond (at least in part) to the electromagnetic wave when exiting the cornea. The corneal refractive aberrations may thus introduce a significant crosstalk over the desired signal that is assumed to impinge on the divider element for determining the objective refraction. Hence, in this case, the measurement of the wavefront analyzer 16 would be based on a confounded (or even false) signal of the electromagnetic wave which would lead to a less accurate (or even false) determination of the objective refraction. However, the inventive concept may enable to actively correct (and thus suppress) the corneal refractive aberrations via the optical element such that the interfering effect of the corneal refractive aberrations may be significantly reduced during measurement.

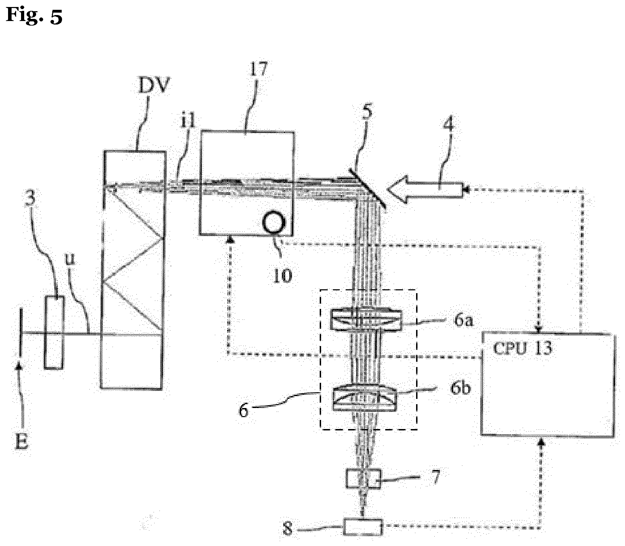

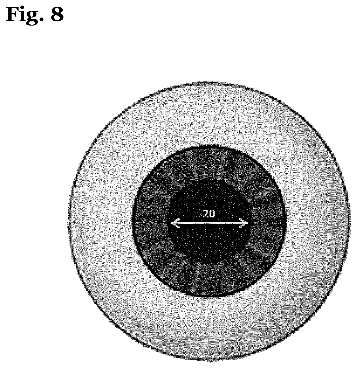

An example of the dynamic pupillometric channel DPC (or DPC unit) is shown in Figure 5. In this example, the eye E of a patient is enlightened by a chamber of illumination 17, that is controlled by a control unit 13. Control unit 13 may be the same as outlined with reference to Fig. 2. The variation of the level of light projected onto the eye E of the patient may determine the change in the diameter of the iris diaphragm 20 (cf. Fig. 8). The illumination chamber 17 may emit light at one or more wavelengths in the visible spectrum, preferably white light. The illumination chamber may comprise a lighting system of LEDs that can generate a diffused and/or concentrated lighting stimulus on the eye of the patient E. The illumination chamber may be preferably equipped with a sensor/ photodiode 10 to measure the light intensity inside the chamber. The apparatus may comprise one or more LEDs 3 (also shown as optional in Fig. 2) that may function to image the iris diaphragm of the eye. The one or more LEDs may emit light in a first infrared wavelength, for example, between 700 nm and 800 nm, wherein the one or more LEDs 3 may be located between the eye E and the optical divider DV to illuminate the eye E of the patient to allow to the sensor 8 to capture the image of the iris diaphragm. Notably, the LEDs 3 and their emitted first infrared wavelength should not influence the motility of the iris diaphragm such that the iris diaphragm is not sensitive to radiation in the first infrared wavelength to enable a steady imaging of the iris diaphragm. The image of the eye E, enlightened by the LEDs 3, may go through the optical divider DV (which may be the same as that shown in Fig. 2) and the illumination chamber 17 to reach the sensor 8 (e.g., a CCD and/or CMOS sensor). The sensor 8 may only acquire the light radiation emitted by LEDs 3 and reflected by the eye E. The sensor 8 may be connected to the control unit 13. The control unit 13 may collect the signal detected by the sensor 8 and extract the herein mentioned measurement data of the diameter of the iris diaphragm 20, and consequently adjust the level of light in the illumination chamber 17 in a way that the iris diaphragm diameter 20 is equal to a predefined value with a tolerance to be selectively set, for example, via a man/machine interface. In combination to said control of the diameter of the iris diaphragm 20, the ORC and/or the SRC may execute their optometric measurements on the eye E with a predetermined iris diaphragm.

In an example, the illumination chamber may be equipped with a photodiode 10, positioned internally in the illumination chamber, and connected to the control unit 13, to measure the light intensity. This may enable to detect the level of light of the illumination chamber 17 during the control exercised on the iris diaphragm diameter 20. In an example, upstream of the sensor 8, with respect to the direction of the light radiation that originates from the eye E towards the sensor 8, a telecentric lens 6 may be installed (e.g., in form of a focal doublet, implemented by the optics 6a and 6b), to allow the focusing of the image of the iris diaphragm onto the sensor 8 (e.g., to avoid magnification errors of the image). Between the telecentric lens 6 and sensor 8 a narrow-band filter 7 may be positioned to allow the selective passage of the first infrared wavelength. This may allow for a more accurate extraction of the diameter of the iris diaphragm 20 by means of the control unit (13).

In an example, the control unit 13 of the DPC may be configured to enable a variation of the quantity of light inside the illumination chamber 17, to vary the diameter of the iris diaphragm 20 to a set value by an automatic or manual procedure (e.g., selectable by an operator). The method of controlling the diameter of the iris diaphragm 20 by the DPC, which is operated by the control unit 13 may comprise the following steps:
- predefining, via an input using a man/machine interface, of the desired reference value of the diameter of the iris diaphragm 20, to execute refractometric measurement (e.g., an objective refraction and/or subjective refraction and/or quality of vision measurement);
- acquisition, via a sensor 8, of at least one image of the iris diaphragm in a preselected condition, via a man/machine interface, of the lighting of the illumination chamber 17;
- extraction of the value of the diameter of the iris diaphragm 20 in said preselected condition of lighting of the illumination chamber 17.

The extracted diameter and the predefined reference value may then be compared.

If they match to within a threshold that may be predefined or at the operator's discretion, the method may end. Otherwise, the operator and/or the DPC may automatically adjust the lighting condition to move closer to the predefined reference value. The acquisition and extraction steps may then be repeated, as well as the comparing step.

Subsequently, the functionality that can be implemented from the herein mentioned DPC, when equipped with the photodiode 10 is described. In an example, the control unit 13 may be configured, not only to control the dimension of the diameter of the iris diaphragm 20 during the execution of objective refraction and/or subjective refraction and/or quality of vision measurements. The control unit 13 may also be configured to execute measurements of the diameter of the iris diaphragm 20 for several, numerable and possibly predefined, values of illumination of the chamber 17. In this scenario, the photodiode 10, by means of the control unit 13, may allow to set different predefined values of the lighting of the chamber 17 and, to correspondingly determine the size of the diameter of the iris diaphragm 20. Said illumination values may be determined in the range that varies between the photopic condition of the patient, that is in the condition of maximum environmental lighting (for example 100.000 lux), and the scotopic condition, that is absolute darkness (for example 0 lux). In an example, the control unit 13 may be suitable to allow multiple acquisitions (that may be repeated) of the value of the diameter of the iris diaphragm 20, with the purpose to detect the dynamic stabilization of the iris diaphragm for each predefined condition of lighting and therefore, to allow the detection of the average diameter of the iris diaphragm 20 for each predefined condition of illumination. Notably, the DPC may enable the detection of the iris diaphragm average and/or maximum and/or minimum diameter, as well as related angles of orientation with respect to a predefined reference system. The DPC may comprise an optical divider 5, which may be totally reflective in the first infrared wavelength that images the eye E by means of the LEDs 3 such that the image is transmitted via the first infrared wavelength onto the sensor 8. However, the optical divider 5 may be totally transparent in a second infrared wavelength, for example, for a wavelength between 800 nm and 900 nm, to allow the injection of the second infrared wavelength onto the retina 22 of the eye E which may be focused thereon. The laser diode 4 may be the emitter of the second infrared wavelength which may be guided onto the retina 22 of the eye E to allow the execution the objective refractometric measurement by means of the ORC unit as described herein. The laser diode 4 may also comprise any other light source that is suitable to execute the objective refractometric measurement (e.g., a light emitting diode, any other types of laser light sources) and may thus not be limited to a laser diode, as such.

Coming back to the ORC unit of Fig. 2 it should be noted that the filter 15 in front of the wavefront analyzer 16 is a pass-band filter that allows the exclusive transmission of the second infrared wavelength (e.g., emitted from the laser diode 4 of the DPC unit) into the wavefront analyser 16.

Subsequently, an interplay of the DPC unit, the ORC unit and the SRC unit of the exemplary apparatus is explained. In an example, the optical divider 11 shown in Fig. 2 may separate the optical path i2 of the ORC and the optical path i3 of the SRC. The optical divider 11 may be totally reflective to light that is used for the optical path i3 of the SRC for performing a subjective refraction, and totally transparent in the second infrared wavelength (i.e., light emitted from the laser diode 4), to allow the transmission of the second infrared wavelength into the wavefront analyzer 16, to perform the objective refractometric analysis, as per the optical path i2 of the ORC. This example should demonstrate that the subdivision between channels shown in Figure 1 is an example to illustrate a schematic model of the invention. The laser diode 4 that emits an electromagnetic wave (in the second infrared wavelength) that is to be reflected at the retina of the eye may also be comprised in the ORC unit and/or the SRC unit.

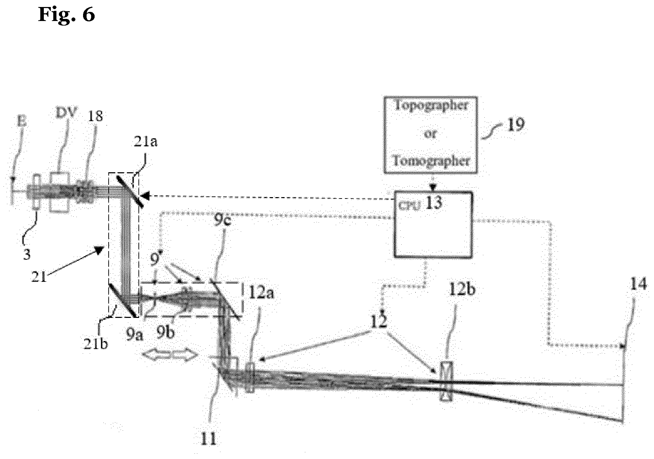

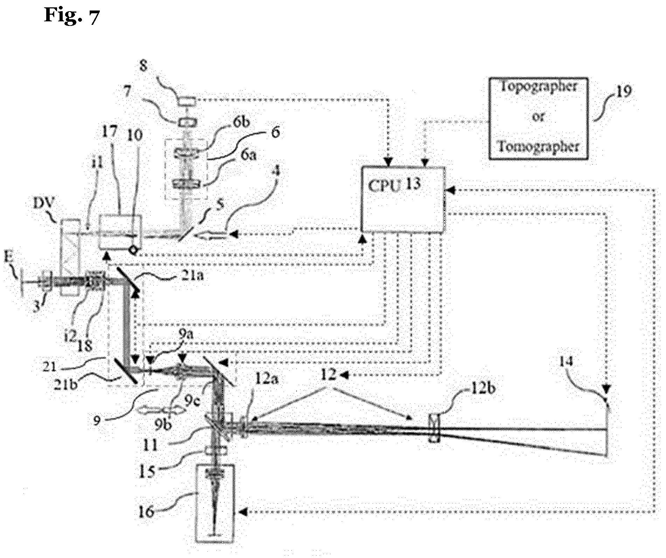

Furthermore, Fig. 6 shows details of an exemplary SRC unit. It should be noted that in the modular example of the apparatus, the DPC, the SRC and the ORC can be combined as shown in Fig. 7. The SRC unit may share in this example a part of its optical path i3 with a part of the optical path i2 of the ORC. While the invention also comprises an SRC unit as a separate apparatus, for the sake of simplicity, its details will be described with reference to Fig. 7.

The SRC unit may implement means and functions to execute the subjective refraction determination of the eye E and the determination of the quality of vision of the eye E. As a main component to perform the subjective refraction determination, the SRC unit may comprise the display 14 that may display various signs, shapes, images. In other examples, the SRC unit may not comprise a display. The optical path of the SRC unit may be connected to a part of the ORC unit via the optical divider 11 which can be seen in Fig. 7. The optical divider 11 may separate the optical path i2 of the ORC and the optical path i3 of the SRC. The optical divider 11 may, for example, be totally reflective in the white light, to allow the reflection into the eye E of the image of the display 14 that is used to perform the analysis of the subjective refraction. The optical divider 11 may be totally transparent in the second infrared wavelength, to allow the transmission of the second infrared wavelength into the wavefront analyzer 16, to perform the objective refractometric analysis, as per the optical path i2 of the ORC.

As seen in Fig. 7 for an exemplary apparatus, the SRC unit may share with the ORC unit, the focus lens 18, the group of tilting mirrors 21, the optical correction group 9 and the optical divider 11 described herein. Hence, the light that is coupled into the eye E over the SRC unit (e.g., an image of the display 14) may be adapted via the tilting mirrors 21, as well as the optical correction group 9 (which may be the parts of the optical element that is configured to compensate an aberration of the eye). The visual perception of the patient looking into the subjective refraction channel SRC may thus be adapted which enables performing a subjective refraction determination. In another example, the ORC unit and SRC unit can also be implemented independently from each other.

The subjective refraction of the eye E and the quality of vision of the eye E may be determined via the SRC unit in far distance vision condition and/or in accommodated near distance vision condition by means of the remoter lens 12 (which may comprise an afocal doublet, implemented by the optics 12a and 12b) to stimulate the lens 23 of the eye E for far distance vision or to accommodate for near distance vision, preferably at a preselected diameter of the iris diaphragm 20. The display 14 may project the image of the display on the remoter lens 12. The image projected by the display 14 may be adapted to simulate the vision of the display 14 to an eye E both in "far vision" and "near vision" conditions. The apparatus may thus be configured to adapt an image the eye perceives of the display 14 to a remote location, as well as a closer location to inflict the "far vision" and/or "near vision" conditions. The stimulation with respect to a "far vision" or "near vision" may also be performed for the ORC unit when performing an objective refraction determination (since also for performing the objective refraction the display 14 may be used to have the patient focus on a certain displayed object).

The control unit 13 (and/or an operator communicating to the control unit 13 via a man/machine interface) may iteratively control the display 14 and the optical correction group 9, according to the feedback of the patient, to determine the subjective refraction and the quality of vision of an eye E both in "far vision" and "near vision" conditions. The subjective refraction examination may be performed at a preselected diameter of the iris diaphragm 20 via the mechanism of the DPC unit described herein.

The evaluation of the quality of vision of the patient (e.g., in conditions of controlled diameter of the iris diaphragm 20) can be executed by projecting one or more tests on the display 14 which may permit to evaluate interactively, as a function of the feedback of the patient in relation to the test, the quality of vision in conditions of controlled diameter of the iris diaphragm 20. The test displayed on the display 14 may comprise a "Contrast Sensitivity Chart", a visual acuity test, etc.

For illustrative purposes Fig. 8 displays an iris diaphragm diameter that may be controlled for the ORC and/or SRC measurements as described herein.

The present invention can be realized very conveniently via a computer program that includes coding means for the realization of one or more steps of the method when this program is executed on a computer. Therefore, it is intended that the scope of protection is extended to said program for computers and additionally to means readable by computers that include a recorded message, said means readable with a computer include means of coding of the program for the realization of one or more steps of the method, when said program is executed on a computer.

Some realization variants to the non-limiting example described are possible, without exiting the scope of the present invention.

From the description mentioned above, the expert in the field can realize the object of the invention without introducing further construction details. The elements and the characteristics illustrated in the preferred different forms of realization can be combined with each other without exiting the scope of the present application. What is described with reference to the state of the art, unless specifically excluded, must be considered in combination with the characteristics of the present invention, forming an integral part of the present invention.

## Claims

1. Apparatus for determining an objective refraction of an eye, comprising an optical element configured to compensate an aberration of the eye.

2. Apparatus according to claim 1, wherein the optical element is configured to compensate a third order and/or an even higher order aberration of the eye.

3. Apparatus according to any of claims 1-2, wherein the optical element is configured to compensate a first order aberration and/or a second order aberration of the eye.

4. Apparatus according to any of claims 1-3, wherein the optical element is configured to compensate an aberration of a cornea of the eye.

5. Apparatus according to claim 4, wherein the optical element is configured to compensate the aberration of the cornea based at least in part on a topographic and/or a tomographic information of the cornea.

6. Apparatus according to any of claims 1-5, wherein the optical element comprises an adaptive optics and/or an adaptive mirror.

7. Apparatus according to any of claims 1-6, further comprising:
a detector for detecting an electromagnetic wave reflected by the eye;
a divider element positioned between the detector and the eye, wherein the divider element is configured such that the electromagnetic wave is divided into a plurality of wave components.

8. Apparatus according to claim 7, wherein the divider element is configured such that at least one of the wave components comprises a portion of the electromagnetic wave originating from a corresponding region on a surface of a cornea of the eye, and wherein the optical element is configured to compensate the aberration such as to reduce an amount of the electromagnetic wave in the at least one of the wave components that originates from a different region of the cornea; and/or
such as to increase the portion of the electromagnetic wave originating from the corresponding region of the cornea comprised by the at least one of the wave components.

9. Apparatus according to claim 8, wherein the optical element is configured to compensate the aberration such that the at least one of the wave components has a one-to-one correspondence to the corresponding region of the cornea.

10. Apparatus according to any of claims 1-9, wherein the apparatus further comprises an optical path for determining a subjective refraction associated with the eye, wherein the optical element is arranged in the optical path.

11. Apparatus according to any of claims 1-10, wherein the apparatus further comprises means for controlling a diameter of an iris diaphragm of the eye by illuminating the iris of the eye.

12. Method for determining an objective refraction of an eye, comprising:
compensating an aberration of the eye by an optical element.

13. Method according to claim 12, further comprising:
obtaining and/or measuring topographic and/or a tomographic information of a cornea of the eye,
wherein the compensating comprises compensating an aberration of the cornea based on the topographic and/or a tomographic information.

14. Method according to claim 13, wherein the compensating further comprises compensating a first order, a second order, a third order and/or even a higher order aberration of the eye.

15. Computer program comprising instructions that when executed by a computer or an apparatus according to any of claims 1-11 causes the computer or the apparatus to perform a method according to any of claims 12-14.
